# EUROPÄISCHE PATENTSCHRIFT

(11) **EP 2 123 323 B1**
(45) Veröffentlichungstag und Bekanntmachung des Hinweises auf die Patenterteilung: **03.10.2012**
(21) Anmeldenummer: 09158895.4
(22) Anmeldetag: 28.04.2009
(51) Int. Cl.: A61N 1/05

(54) **Implantierbare Elektrodenleitung**
Implantable electrode lead
Fil d'électrode implantable

(30) Priorität: 23.05.2008 DE 102008024924
(43) Veröffentlichungstag der Anmeldung: 25.11.2009
(73) Patentinhaber: Biotronik CRM Patent AG, 6341 Baar (CH)
(72) Erfinder: Geistert, Wolfgang, 79618, Rheinfelden (DE); Franke, Ulrich, 13591, Berlin (DE)
(74) Vertreter: Lindner-Vogt, Karin L.

(56) Entgegenhaltungen:
- EP-A1- 0 546 414
- WO-A1-2007/022180
- DE-T2- 69 627 790
- US-A- 4 913 164
- US-A- 4 957 118
- US-A1- 2007 021 813
- US-A1- 2007 073 351

## Beschreibung

Die Erfindung betrifft eine Vorrichtung zur Befestigung von vorübergehend oder dauerhaft implantierbaren medizinischen Geräten mit einem Basiskörper, mindestens einem flexiblen fingerförmigen Verankerungsmittel und mindestens einer flexiblen undehnbaren Vorrichtung zum Zurückziehen jeweils eines zugeordneten Verankerungsmittels.

In einem weiteren Aspekt betrifft die Erfindung eine Elektrodenleitung mit einem Schaft und einer Vorrichtung zur Befestigung von vorübergehend oder dauerhaft implantierbaren medizinischen Geräten.

Weiterhin betrifft die Erfindung ein System zum Überführen eines dehnbaren Abschnittes einer erfindungsgemäßen Vorrichtung oder Elektrodenleitung von einem ersten ungedehnten in einen zweiten gedehnten, gegenüber dem ersten Zustand verlängerten Zustand.

Und abschließend betrifft die Erfindung ein Verfahren zum Bewegen des mindestens einen Verankerungsmittels der erfindungsgemäßen Vorrichtung oder Elektrode in Richtung eines Basiskörpers.

Solche Elektrodenleitungen sind im Stand der Technik beschrieben. So beschreibt die US 4,913,164 abklappbare passive Verankerungsvorrichtungen, welche fest mit einer Art Führungsdraht verbundene Vorrichtung zum Zurückziehen dieser Tines aufweist. Durch Ausübung einer Bewegung auf diese Art Führungsdraht können die Tines angeklappt werden, um ein besseres Einführen zu erleichtern. Auch die US 4,957,118 beschreibt solche Vorrichtungen zum Zurückziehen von Tines einer Elektrodenleitung. Diese Vorrichtungen weisen alle den Nachteil auf, dass ein technisch sehr komplexer Aufbau eine leichte Produktion nicht ermöglicht. Auch seitens der Zuverlässigkeit bestehen gravierende Nachteile.

Vorübergehend oder dauerhaft implantierbare medizinische Geräte müssen im Körper zwangsweise befestigt werden, damit die Lage stabil bleibt, um genaue Messergebnisse zu liefern oder schädliche Auswirkungen auf den Körper zu verhindern. Dabei stellt es eine große Herausforderung dar, ein solches Gerät am richtigen Ort in einem menschlichen oder tierischen Körper zu platzieren. Beispielsweise kann es sich nach dem Implantieren eines Messsensors herausstellen, dass der Implantationsort nicht geeignet ist, genaue Messergebnisse aufzunehmen. Dann muss eine Reposition an einem geeigneteren Ort erfolgen.

Es hat sich herausgestellt, dass sich die Reposition oder Explantation von vorübergehend oder dauerhaft implantierbaren medizinischen Geräten als schwierig erweist. Dies ist vor allem bei so genannten passiv fixierbaren implantierbaren medizinischen Geräten ein Problem, da hier Verankerungsmittel vom Basiskörper abstehen. Diese Verankerungsmittel bewirken zwar eine gute bis sehr gute Verankerung des implantierbaren medizinischen Gerätes, jedoch sind solche passiven Verankerungsmittel nur unter großen Umständen repositionierbar bzw. explantierbar.

Eine geeignete Lösung zeigt das Dokument EP 0 546 414 A1, welche eine Vorrichtung zur Befestigung von vorübergehend oder dauerhaft implantierbaren medizinischen Geräten. Die dort gezeigte Vorrichtung umfasst einen Basiskörper mit einem ersten Ende, einem zweiten Ende und einem zwischen dem ersten und zweiten Ende befindlichen flexiblen und dehnbaren Abschnitt, der von einem ersten ungedehnten in einen zweiten gedehnten und gegenüber dem ersten Zustand verlängerten Zustand überführbar ist und bei dessen Überführung in den zweiten Zustand der ein daran angebrachtes Verankerungsmittel, mit dessen Hilfe die Befestigung im Gewebe erfolgt, abklappt, so dass die Verankerungsmittel am Basiskörper nahezu anliegen.

Ausgehend vom Stand der Technik liegt der vorliegenden Erfindung die Aufgabe zugrunde, die genannten Nachteile zu beheben, indem eine sichere, leicht fixierbare und wieder leicht lösbare Verankerung von vorübergehend oder dauerhaft implantierbaren medizinischen Geräten ermöglicht wird, die leicht repositioniert und/oder explantiert werden kann. Weiterhin sollen die weiteren Nachteile des Stands der Technik bezüglich Zuverlässigkeit, Herstellbarkeit und Produktion behoben werden.

Diese Aufgabe wird mit Anspruch 1 gelöst.

Die Vorrichtung zur Befestigung von vorübergehend oder dauerhaft implantierbaren medizinischen Geräten umfasst einen Basiskörper mit einem ersten und einem zweiten Ende, und einem zwischen dem ersten und zweiten Ende befindlichen flexiblen und dehnbaren Abschnitt, der von einem ersten ungedehnten in einen zweiten gedehnten und gegenüber dem ersten Zustand verlängerten Zustand überführbar ist, ein das zweite Ende des Basiskörpers bildendes, am flexiblen und dehnbaren Abschnitt fest angebrachtes, unflexibles und undehnbares erstes Widerlager (3), mindestens ein erstes flexibles fingerförmiges Verankerungsmittel das vom genannten Basiskörper absteht und an einem Montagepunkt am ersten Widerlager des Basiskörpers angebracht ist, und mindestens eine erste flexible Vorrichtung zum Zurückziehen jeweils eines zugeordneten ersten Verankerungsmittels, wobei die Vorrichtung fest an einem ersten Befestigungspunkt mit dem flexiblen und dehnbaren Abschnitt des Basiskörpers und mit dem zugeordneten ersten Verankerungsmittel verbunden ist, wobei sich der erste Befestigungspunkt vom Montagepunkt entfernt und wobei sich beim Überführen des Abschnittes vom ersten in den zweiten Zustand die erste Vorrichtung zum Zurückziehen nicht ausdehnt, so dass das zugeordnete erste Verankerungsmittel in Richtung des Basiskörpers bewegt wird.

Erfindungsgemäß ist der flexible und dehnbare Abschnitt und das erste Verankerungsmittel aus demselben dehnbaren Material einstückig hergestellt, wobei das Material das erste Widerlager (6) mindestens abschnittsweise umhüllt.

Die erfindungsgemäße Bewegung der Verankerungsmittel in Richtung des Basiskörpers bewirkt, dass die angeklappten Verankerungsmittel nicht am Köpergewebe hängen bleiben und Verletzungen herbeiführen. Die Verankerungsmittel können so lange angeklappt bleiben, bis die Reposition an einem geeigneteren Implantationsort abgeschlossen ist. Dann können die Verankerungsmittel ganz leicht wieder aufgeklappt werden. Somit besteht der Vorteil neben der erleichterten Reposition oder Explantation in einer leichten Wiederverankerung oder Fixierung durch die Verankerungsmittel an einem geeigneteren Implantationsort.

Weiterhin hat es sich als vorteilhaft herausgestellt, dass die Verlängerung des dehnbaren Abschnittes von einem ersten ungedehnten Zustand zu einem gedehnten zweiten Zustands positive Auswirkung auf das Lösen der Fixierung hat. Durch die Verlängerung des dehnbaren Abschnittes und damit des Basiskörpers bei gleichzeitigem Anklappen der Verankerungsmittel enthakt sich das vorher eingehakte Verankerungsmittel wesentlich leichter aus dem Gewebe.

Unter einem vorübergehend oder dauerhaft implantierbaren medizinischen Gerät wird im erfindungsgemäßen Sinne jede Vorrichtung verstanden, die in den Körper eingeführt werden können, um dort diagnostische oder therapeutische Wirkung zu entfalten. Dies können elektrisch aktive Implantate wie Herzschrittmacher, Defibrillatoren, Kardioverter oder Nervenstimulatoren sein. Umfasst sind auch Vorrichtungen, die sich in der Peripherie solcher elektrisch aktiven Implantate befinden, wie Elektrodenleitungen, die in ein Körperorgan führen können wie intrakardiale Elektrodenleitungen, epikardiale Elektrodenleitungen, Elektroden zur Nervenstimulation oder so genannter "Deep-Brain-Stimulation". Zu solchen Geräten zählen erfindungsgemäß aber auch Sensoren zur Messung von physiologischen Körpersignalen wie Blutdruck oder Sauerstoffsättigung, welche in Zusammenwirken mit einem weiter oben genannten elektrisch aktiven Implantat oder aber mit einem Sender/Empfänger zur Übertragung der gemessenen Daten an ein externes Gerät stehen. Umfasst sind erfindungsgemäß auch Sender-/Empfängereinheiten zum Übertragen oder Senden von Daten in oder aus dem Körper, wie beispielsweise Antennen zur Nah- oder Fernfeldtelemetrie. Erfindungsgemäß kann es sich bei einem solchen implantierbaren Gerät auch um Implantate zur vaskulären Intervention handeln wie Stents, Bypässe, Coils oder Medikamentendepots.

Als Basiskörper im erfindungsgemäßen Sinn sind sämtliche dreidimensionale Körperformen vorstellbar. So weist eine tubuläre, schlauchförmige Form keine negativen Effekte in Bezug auf die Strömung des Blutflusses in einem Gefäß, auf. Vorstellbar sind auch quaderförmige, halbrunde, kugel- oder tropfenförmige Basiskörper. Der Basiskörper kann jedoch auch eine anatomische Form aufweisen, der sich beispielsweise in seiner Form der Körperhöhlung anpasst oder angepasst wird, in die er implantiert wird.

Bevorzugt weist die erste Vorrichtung zum Zurückziehen einen zweiten Befestigungspunkt an dem dem Basiskörper abgewandten Ende des ersten/oder zweiten Verankerungsmittels auf, wobei zwischen dem ersten und zweiten Befestigungspunkt Mittel vorgesehen sind, die die Dehnung zwischen den Befestigungspunkten verhindern oder verringern. Diese Mittel können Stahlseile oder andere geeignete undehnbare Materialien/Elemente sein.

Es hat sich als vorteilhaft herausgestellt, wenn der Basiskörper einer Vorrichtung zur Befestigung eines vorübergehend oder dauerhaft implantierbaren Geräts flexibel ausgelegt ist, da so die natürlichen Bewegungen (beispielsweise die Peristaltik) von Körpergefäßen besser ausgeglichen werden und somit weniger zu Reizungen im Gewebe führen, an dem die Vorrichtung und damit das implantierbare Gerät befestigt ist. Denn diese Reizungen rufen entzündliche Veränderungen am Gewebe hervor und können im Falle einer in einem Blutgefäß implantierten Vorrichtung schlimmstenfalls zu einem Gefäßverschluss führen, was lätale Auswirkungen haben kann.

Erfindungsgemäß ist der dehnbare Abschnitt des Basiskörpers so dehnbar ausgelegt, dass er einen ersten ungedehnten Zustand und einen zweiten gedehnten Zustand aufweist. Dabei sind die Materialeigenschaften des flexiblen und dehnbaren Abschnittes so gewählt, dass zwischen diesen beiden ersten und beiden Zuständen beliebig oft gewechselt werden kann, die Vorrichtung zur Befestigung also beliebig oft repositioniert werden kann. Der gedehnte zweite Zustand ist dann erreicht, wenn durch das Zusammenwirken mit der Vorrichtung zum Zurückziehen die Verankerungsmittel am Basiskörper anliegen. Dabei sind die Verankerungsmittel so konstruiert, dass sie so vorgespannt sind, dass sie beim Zurückführen in den ersten ungedehnten Zustand des dehnbaren und flexiblen Abschnittes in die abstehende Ausgangsposition zurückfedern. Dies kann baulich beispielsweise dadurch sichergestellt werden, dass die Basis eines jeden Verankerungsmittels am Montagepunkt verbreitert ist oder jedes Verankerungsmittel beispielsweise konisch vom Montagepunkt aus Richtung dem Basiskörper abgewandten Ende des Verankerungsmittels verläuft.

Als Verankerungsmittel im Sinne der Erfindung gelten unter anderem herkömmliche und gebräuchliche so genannte Tines mit oder ohne einer Vorzugsdrehrichtung, wie sie zur Verankerung kardialer Elektrodenleitungen Verwendung finden. Aus der DE 10 2006 014 698.0 sind solche Tines mit einer Vorzugsdrehrichtung bekannt. Die Schrift wird in seiner kompletten Offenbarung als beinhaltet angesehen.

Im Zusammenhang mit den genannten Ausführungsformen sind die erste und/oder zweite Vorrichtung zum Zurückziehen als dünne, flexible Rippen ausgebildet, welche sich zwischen den jeweiligen ersten und zweiten Befestigungspunkten und den jeweiligen Montagepunkten aufspannen.

In einer bevorzugten Ausgestaltung sind diese flexiblen Rippen auf den Strecken zwischen den jeweiligen ersten Befestigungspunkten und den jeweiligen Montagepunkten und zwischen den jeweiligen zweiten Befestigungspunkten und den jeweiligen Montagepunkten ununterbrochen am Basiskörper bzw. den ersten und/oder zweiten Verankerungsmitteln befestigt. Es sind aber unter dem Begriff der dünnen flexiblen Rippen auch Membrane zu subsumieren - aufgebaut wie die aus der Herpetofauna bekannten "Schwimmhäute".

Eine Schwimmhaut ist die Haut, die zwischen den Zehen oder Fingern schwimmender Tiere ausgebildet ist. Sie erhöht die Effizienz der Schwimmbewegungen, indem sie die Fläche der bewegten Füße oder Hände vergrößert und so eine bessere Übertragung der Muskelkraft auf das Wasser ermöglicht. Schwimmhäute sind elastisch (aus Wikipedia, der freien Enzyklopädie, 16.07.2007; http://de.wikipedia.org/wiki/Schwimmhaut).

In diesem Zusammenhang sind der dehnbare Abschnitt, das erste und/oder zweite Verankerungsmittel und gegebenenfalls zusätzlich die Rippen aus demselben dehnbaren Material einstückig hergestellt und umhüllt das erste und/oder zweite Widerlager mindestens abschnittsweise. Der Abschnitt, der vom ersten und/oder zweiten Widerlager mit demselben dehnbaren Material beschichtet ist, erstreckt sich dabei um den ersten bzw. zweiten Montagepunkt herum. Dieser Abschnitt wird dabei einerseits durch das eine Ende des ersten bzw. zweiten Widerlagers begrenzt, das mit dem flexiblen Abschnitt verbunden ist. Andererseits erstreckt sich dieser Abschnitt soweit in die dem genannten Ende entgegen gesetzte Richtung, dass der erste bzw. zweite Montagepunkt im beschichteten Bereich liegt.

Das mindestens eine erste oder zweite Verankerungsmittel ist vorzugsweise so angeordnet, dass die jeweilige Längsachse der Verankerungsmittel einen veränderlichen Winkel zum Basiskörper aufweist, wobei der Winkel im ersten ungedehnten Zustand flach und im zweiten gedehnten und gegenüber dem ersten Zustand verlängerten Zustand des flexiblen dehnbaren Abschnittes spitz ist.

In einer weiteren Ausführung der erfindungsgemäßen Befestigungsvorrichtung weist der Basiskörper ein zweites am ersten Ende des Basiskörpers fest angebrachtes unflexibles und undehnbares Widerlager auf. In dieser Ausführung umfasst die Befestigungsvorrichtung bevorzugt:
- mindestens ein zweites flexibles fingerförmiges Verankerungsmittel, welches vom Basiskörper absteht und an einem Montagepunkt am zweiten Widerlager angebracht ist, und
- mindestens eine zweite flexible, undehnbare Vorrichtung zum Zurückziehen jeweils eines zugeordneten zweiten Verankerungsmittels, wobei die zweite Vorrichtung fest mit dem flexiblen und dehnbaren Abschnitt des Basiskörpers und mit dem zugeordneten zweiten Verankerungsmittel verbunden ist.

Diese genannten ersten und/oder zweiten Widerlager können in einer besonderen Ausgestaltung ein erstes und/oder zweites Fixierungsmittel umfassen.

Diese Fixierungsmittel erleichtern es, beispielsweise mittels Hilfsmittel Kräfte auszuüben, die in Ihrer Wirkung den dehnbaren flexiblen Abschnitt von einem ersten ungedehnten in einen zweiten gedehnten und gegenüber dem ersten Zustand verlängerten Zustand überführen. Als Fixierungsmittel im Sinne der Erfindung werden Einrichtungen angesehen, die es ermöglichen, einen anderen Gegenstand an der Befestigungsvorrichtung zu fixieren mit dem Ziel, Kräfte zu übertragen oder andere Wechselwirkungen zwischen diesem Gegenstand und der Befestigungsvorrichtung auszuführen. Die Fixierungsmittel sind dabei ausnahmslos an der Befestigungsvorrichtung fest angebracht. Geeignete Fixierungsmittel sind in diesem Sinne Anschläge, Krägen oder Mittel zum Einhaken von anderen Gegenständen oder jeder andere geeignete Mechanismus.

Der Vorteil dieses Aspektes der Erfindung besteht darin, dass durch Hinzunahme nur eines einfachen und robusten Bauteils die erfindungsgemäße Befestigungsvorrichtung geringfügig verändert werden muss, um einen einfachen Aufbau zu erhalten, der es ermöglicht, ein Anklappen der flexiblen Vorrichtung an den Schaft zu bewirken. Besonders vorteilhaft ist dieser einfache Aufbau, weil dadurch die aus dem Stand der Technik bekannten aufwändigen Mechanismen vermieden werden. Diese Mechanismen haben durch ihre Dreh- und/oder Gleitlager Zuverlässigkeits- und Dichtigkeitsprobleme. Weiterhin lässt sich eine solche Vorrichtung sehr leicht und mit wenigen Prozessen herstellen.

In einer weiteren Ausführung umfasst der Basiskörper der Befestigungsvorrichtung ein Lumen, welches eine Öffnung im ersten Ende aufweist, sich bis zum zweiten Ende erstreckt und vorzugsweise eine weitere Öffnung im zweiten Ende des Basiskörpers aufweist. In einer weiteren Ausgestaltung der Ausführung umfasst die Öffnung am ersten und/oder zweiten Ende des Basiskörpers ein Dichtelement, vorzugsweise einen Lippendichtring. Dabei kann in einer besonders bevorzugten Ausgestaltung das weiter oben beschriebene erste Fixierungsmittel in der Öffnung im zweiten Ende angeordnet sein und das zweite weiter oben beschriebene Fixierungsmittel vorzugsweise in der Öffnung des ersten Endes.

Ein Lumen ist im Zusammenhang mit der Erfindung von Vorteil, weil dieses Lumen einerseits als Medikamentendepot dienen kann, andererseits aber auch zur Aufnahme von elektrisch aktiven oder passiven Bauteilen dienen kann, die die Funktionalität des dauerhaft oder vorübergehend implantierbaren Geräts sicherstellt. Bevorzugt dient dieses Lumen jedoch zur Aufnahme der Fixierungsmittel zur Bedienung der Befestigungsvorrichtung. Das erfindungsgemäße Lumen ist erfindungsgemäß ein Hohlraum im Inneren der Vorrichtung, das jede dreidimensionale Form annehmen kann, die dazu vorgesehen ist, die Funktionalität des dauerhaft oder vorübergehend implantierbaren Geräts sicherzustellen.

In einem weiteren Aspekt liegt der Erfindung die Aufgabe zugrunde, die Elektrodenleitung aus dem Stand der Technik so weiterzuentwickeln, dass sie einen einfachen Aufbau aufweist und geeignet ist, eine Reposition und/oder Explantation leicht zu ermöglichen.

Diese Aufgabe wird durch Anspruch 10 gelöst.

Die erfindungsgemäße Elektrodenleitung umfasst:
- einen lang gestreckten Schaft mit einem proximalen und einem distalen Ende,
- eine am distalen Ende des Schaftes befestigte erfindungsgemäße Befestigungsvorrichtung, wobei das Basiskörper eine lang gestreckte, schaftartige Form aufweist und das erste Ende mit dem distalen Ende des Schaftes fest verbunden ist,
- mindestens einer elektrisch aktiven Fläche am ersten Widerlager und/oder zweiten Widerlager, und
- mindestens einem elektrischen Leiter, der sich vom proximalen Ende des Schaftes über das distale Ende des Schaftes hinaus bis zu den elektrisch aktiven Flächen erstreckt.

Unter einem Schaft im erfindungsgemäßen Sinne wird eine lang gestreckte Struktur mit einer Längsachse und einem senkrecht zur Längsachse liegenden runden oder elliptischen Querschnitt verstanden. Die Länge - gemessen entlang der Längsachse - übersteigt dabei ein Vielfaches die der Querachsen, die den Querschnitt beschreiben. Der Schaft umfasst dabei einen flexiblen, mehr oder weniger dehnbaren Grundkörper, der nach außen von einer dielektrischen gleitfähigen Schicht gebildet wird. Diese Schicht ist vorzugsweise Polyurethan oder ein Polyurethan-Silikon-Copolymer. Zusätzlich kann der Schaft mindestens ein helixfederförmiges konzentrisch um die Längsachse verlaufendes Stabilisierungselement umfassen, das in die dielektrische gleitfähige Schicht außen eingegossen ist. Die Feder kann auch als elektrischer Leiter dienen, der am proximalen Ende vorzugsweise über einen genormten Stecker (beispielsweise IS-4, DF-1, IS-1) mit einem elektrisch aktiven Implantat elektrisch leitend verbunden ist (und der Signale von dem Implantat übertragen kann). Der elektrische Leiter kann auch ein eigenständiges Bauteil sein.

Unter einer lang gestreckten schaftartigen Form des Basiskörpers wird ebenfalls eine lang gestreckte Struktur mit einer Längsachse und einem senkrecht zur Längsachse liegenden runden oder elliptischen Querschnitt verstanden. Die Länge - gemessen entlang der Längsachse - übersteigt dabei die der Querachsen, die den Querschnitt beschreiben. Der Schaft umfasst dabei einen flexiblen, mehr oder weniger dehnbaren Grundkörper, der nach außen von einer dielektrischen gleitfähigen Schicht gebildet wird. Der Querschnitt der schaftartigen Form des Basiskörpers kann dabei dieselbe oder eine andere Form aufweisen wie der Schaft.

Der Vorteil der leichten Reposition bzw. Explantation kommt vor allem bei intrakardialen Elektrodenleitungen mit passiven Fixierungen zum Tragen. Die Innenwand des Vorhofes bzw. der Herzkammer ist mit dem Trabekelwerk ausgekleidet. Das ist ein Netzwerk von bälkchenartigem Herzmuskelgewebe. In dieser Struktur können sich die Verankerungsmittel leicht verhaken. Dabei kann es bei einer Explantation bzw. Reposition zu teilweise schweren Verletzungen kommen. Somit bewirkt das erfindungsgemäße Anklappen der abstehenden fingerförmigen Verankerungsmittel eine Verringerung des Verletzungsrisikos und eine Erleichterung der Reposition.

Noch deutlicher wirken sich die erfindungsgemäßen Verbesserungen bei schon implantierten intrakardialen Elektrodenleitungen aus, die aufgrund eines Defektes ausgetauscht und explantiert werden müssen. Solche Elektrodenleitungen sind aufgrund natürlicher Prozesse festgewachsen. Somit bildet sich bei herkömmlichen Elektroden aus dem Stand der Technik ein fester Verbund zwischen dem Herzgewebe und den Verbindungsmitteln einer passiven Elektrode. Solche Elektroden sind in der Praxis nicht mehr einfach zu explantieren. Eine Operation am offenen Herzen ist unweigerlich nötig.

Die erfindungsgemäße Elektrode hat nun den Vorteil, dass durch die Zusammenwirkung der Längenveränderung des Basiskörpers der Befestigungsvorrichtung mit dem Anklappen der Verankerungsmittel eine Lockerung des Gewebes eintritt und somit die Explantation ohne große Umstände mit herkömmlichen kardiologischen Mitteln möglich ist.

In diesem Zusammenhang weist der Schaft der Elektrodenleitung an seinem proximalen Ende einen Anschluss auf, der dazu dient, den mindestens einen elektrischen Leiter mit einem elektrisch aktiven Implantat zu verbinden. Vorzugsweise ist der elektrische Anschluss ein Stecker, welcher besonders bevorzugt einem genormten Stecker der Form IS-1 und/oder DF-1, oder IS-4 entspricht.

In diesem Zusammenhang ist die erfindungsgemäße Elektrodenleitung durch einen Schaft gekennzeichnet, der weiterhin ein Lumen umfasst, welches sich vom proximalen Ende bis in das distale Ende des Schaftes erstreckt und im proximalen und distalen Ende eine Öffnung bildet, wobei die Öffnung im distalen Ende und die Öffnung des Basiskörpers verbunden sind derart, dass Führungsdraht und/oder Mandrin geführt werden kann, um die Elektrodenleitung zu führen und/oder den flexiblen dehnbaren Abschnitt zu strecken und/oder zu dehnen.

In diesem Zusammenhang weist der Schaft und/oder flexible dehnbare Abschnitte der erfindungsgemäßen Elektrodenleitung mindestens ein radial-stabiles dehn- und/oder streckbares Stabilisierungselement auf, welches vorzugsweise spiralförmig ausgebildet ist und als elektrischer Leiter dient.

Die erfindungsgemäße Elektrodenleitung ist weiterhin **dadurch gekennzeichnet, dass** der flexible dehnbare Abschnitt des Basiskörpers von mindestens einer flexiblen, dehn - und/oder streckbaren isolierenden Hüllschicht umgeben ist und welche vorzugsweise das erste und/oder zweite Widerlager mindestens abschnittsweise umhüllt und besonders bevorzugt auch das Material der ersten und/oder zweiten Verankerungsmittel und/oder ersten und/oder zweiten Vorrichtungen zum Zurückziehen bildet. Der Abschnitt, der vom ersten und/oder zweiten Widerlager mit demselben dehnbaren Material beschichtet ist, erstreckt sich dabei um den ersten bzw. zweiten Montagepunkt herum. Dieser Abschnitt wird dabei einerseits durch das eine Ende des ersten bzw. zweiten Widerlagers begrenzt, das mit dem flexiblen Abschnitt verbunden ist. Andererseits erstreckt sich dieser Abschnitt soweit in die dem genannten Ende entgegen gesetzte Richtung, dass der erste bzw. zweite Montagepunkt im beschichteten Bereich liegt.

Weiterhin liegt der Erfindung die Aufgabe zugrunde, ein System zur einfachen Anwendung der erfindungsgemäßen Vorrichtung zu bieten.

Diese Aufgabe wird in Anspruch 12 durch ein System zum Überführen des dehnbaren Abschnittes einer erfindungsgemäßen Vorrichtung oder Elektrodenleitung von einem ersten ungedehnten in einen zweiten gedehnten, gegenüber dem ersten Zustand verlängerten Zustand, gelöst, welches ein erstes Fixiermittel im ersten Widerlager und eine Stoßvorrichtung mit einem ersten Gegenanschlag umfasst, wobei der Gegenanschlag unmittelbar in Wechselwirkung mit dem ersten Fixiermittel tritt. In einer besonderen Ausgestaltung ist die Stoßvorrichtung ein Führungsdraht oder Mandrin.

Unter Wechselwirkung im erfindungsgemäßen Sinne ist unter anderem ein Kraftübertrag zu verstehen, wobei die Kraft beispielsweise außerhalb des Körpers von einem Operateur auf die Stoßvorrichtung ausgeübt wird und diese Kraft dann von dieser Stoßvorrichtung auf die Befestigungsvorrichtung mittelbar oder unmittelbar übertragen wird. Diese Kraft ist so gerichtet bzw. dimensioniert, dass sie sich auf den Basiskörper, insbesondere auf den dehnbaren und flexiblen Abschnitt auswirkt, so dass dieser von einem ersten ungedehnten in einen zweiten gedehnten und gegenüber dem ersten gedehnten Zustand eintritt.

Bevorzugt umfasst das erfindungsgemäße System einen zweiten Gegenanschlag, der mittelbar oder unmittelbar in Wechselwirkung mit dem zweiten Fixierungsmittel tritt. In einer besonders bevorzugten Ausgestaltung des erfindungsgemäßen Systems ist der zweite Anschlag ein Klemmmittel, mit dem die Stoßvorrichtung mit dem Schaft der Elektrodenleitung fixiert werden kann und somit in mittelbare Wechselwirkung über den Schaft auf das zweite Fixierungsmittel tritt, um so ein Auflager bezüglich der Wechselwirkung des ersten Gegenanschlags mit dem ersten Fixierungsmittel zu bilden.

Abschließend wird ein Verfahren offenbart, das nicht Teil der Erfindung ist, wobei durch Bewegen des ersten Verankerungsmittels in Richtung des Basiskörpers der dehnbare Abschnitt des Basiskörpers der erfindungsgemäßen Befestigungsvorrichtung oder Elektrodenleitung mit Hilfe des erfindungsgemäßen Systems on einem ersten ungedehnten in einen zweiten gedehnten, gegenüber dem ersten Zustand verlängerten Zustand überführt wird.

Das Verfahren zum Bewegen des ersten Verankerungsmittels in Richtung des Basiskörpers durch Überführen des dehnbaren Abschnittes des Basiskörpers der erfindungsgemäßen Befestigungsvorrichtung oder Elektrodenleitung von einem ersten ungedehnten in einen zweiten gedehnten, gegenüber dem ersten Zustand verlängerten Zustand, mit Hilfe des erfindungsgemäßen Systems umfasst die folgenden Schritte:
- Unmittelbares in Kontakt und damit in Wechselwirkung bringen des ersten Gegenanschlags der Stoßvorrichtung mit dem ersten Fixierungsmittel des ersten Widerlagers,
- Mittelbares oder unmittelbares in Kontakt und damit in Wechselwirkung bringen des zweiten Gegenanschlags der Stoßvorrichtung mit dem zweiten Fixierungsmittel des zweiten Widerlagers,
- Ausüben einer vom zweiten Fixierungsmittel weg führenden Kraft auf die Stoßvorrichtung, welche über den ersten Gegenanschlag auf das erste Fixierungsmittel übertragen wird und das erste Widerlager so auslenkt, dass es sich vom zweiten Widerlager entfernt, um den dehnbaren Abschnitt des Basiskörpers von einem ersten ungedehnten in einen zweiten gedehnten, gegenüber dem ersten Zustand verlängerten Zustand zu überführen.

Im Folgenden wird die Erfindung anhand folgender Zeichnungen erläutert:

Es zeigen:
- Figur 1:: eine erfindungsgemäße Vorrichtung mit einer Darstellung der Bauteile,
- Figur 2:: eine erfindungsgemäße Elektrodenleitung mit einer Darstellung der Bauteile,
- Figur 3:: Ein erfindungsgemäßes System, wobei sich die erfindungsgemäße Befestigungsvorrichtung in einem zweiten gedehnten Zustand befindet.
- Figur 4:: Ein erfindungsgemäßes System, wobei sich der dehnbare und flexible Abschnitt der Elektrodenleitung in einem zweiten gedehnten Zustand befindet.

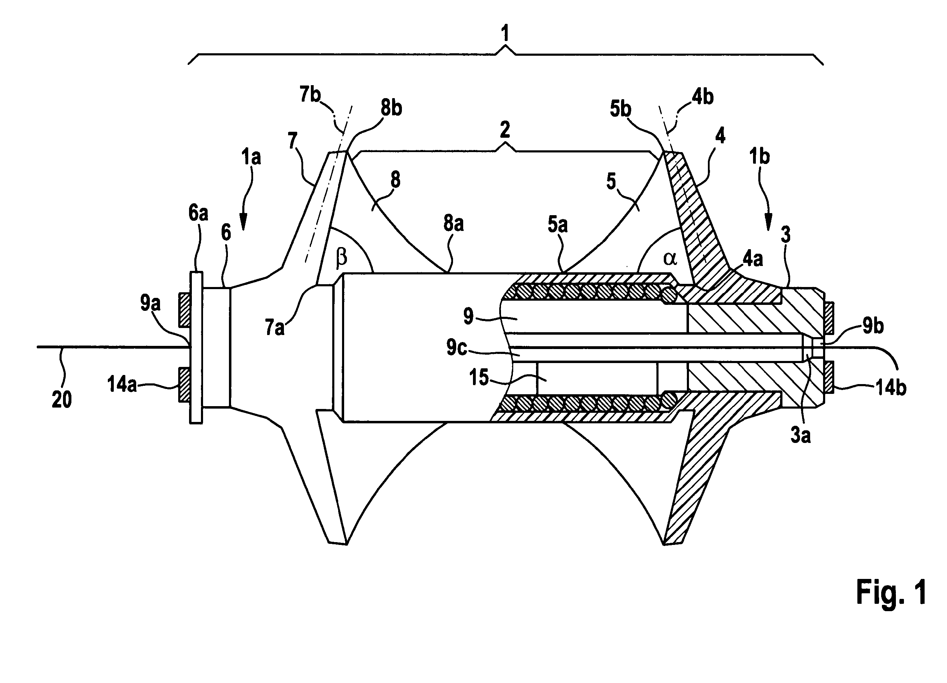

Figur 1 zeigt eine erfindungsgemäße Befestigungsvorrichtung mit einem Basiskörper 1 mit einem ersten Ende 1a und einem zweiten Ende 1b. Der Basiskörper besteht aus einem dehnbaren und flexiblen Abschnitt 2 und einem ersten Widerlager 3 am zweiten Ende 1b und einem zweiten Widerlager 6 am ersten Ende 1a und wird von einem Lumen 9 durchzogen. Das Lumen 9 bildet dabei im ersten Widerlager 3 eine Öffnung 9b und im zweiten Widerlager 6 eine Öffnung 9a. Im Lumen 9 können beispielsweise eine Mikroelektronik zur Auswertung von Daten eines Sensor zur Blutdruckmessung, ein Transceiver zur berührungslosen Datenübertragung der von der Mikroelektronik ausgewerteten Daten an ein extern des Körpers befindliches, nicht dargestelltes Gerät über eine Fern- oder Nahfeldtelemetrie, und eine Stromversorgung angeordnet werden. Diese Bauelemente sind hier der Einfachheit halber zusammenfassend als elektrisch aktive Elemente 15 gekennzeichnet. Der Sensor selbst wird durch zwei Sensorelemente 14a und 14b auf den Widerlagern 6 und 3 gebildet, welche beispielsweise den differenten Umgebungsdruck messen und über nicht dargestellte elektrische Verbindungen an die Mikroelektronik weiterleiten.

Zusätzlich ist im Lumen der Befestigungsvorrichtung ein Führungsdrahtlumen 9c vorgesehen, der die elektrisch aktiven Elemente 15 gegen die Öffnungen 9a und 9b abdichtet. Durch dieses Führungsdrahtlumen kann ein Führungsdraht 20 geschoben werden, der vorher im Körper bzw. in einem Gefäß oder Hohlraum des Körpers an einer Stelle platziert wurde, an dem die Implantation des dauerhaft oder vorübergehend implantierbaren medizinischen Geräts vorgesehen ist. Dadurch, dass das eine Ende des Führungsdrahtes 20 außerhalb des Körpers ist, kann das dauerhaft oder vorübergehend implantierbare medizinische Gerät auf dem Führungsdraht an diesen Ort gebracht werden.

In oder auf den Widerlagern 3 und 6 befinden sich Fixierungsmittel 3a und 6a. In diesem Beispiel wird das Fixierungsmittel 3a durch einen Absatz in der Öffnung 9b gebildet, während das Fixierungsmittel 6a durch einen Kragen gebildet wird.

Des Weiteren weist die Vorrichtung in Figur 1 mindestens ein erstes und mindestens ein zweites flexibles Verankerungsmittel 4 und 7 auf, welche an Montagepunkten 4a und 7a an den ersten und zweiten Widerlagern 3 und 6 befestigt ist. Beide sind so angeordnet ist, dass die jeweilige Längsachse 4b und 7b der Verankerungsmittel 4 und 7 einen veränderlichen Winkel α und β zum Basiskörper 1 aufweisen. Die Winkel sind dabei so angelegt, dass die dem Basiskörper 1 abgewandten Enden der beiden Verankerungsmittel 4 und 7 aufeinander zuweisen. Dabei sind die Verankerungsmittel so konstruiert, dass sie so vorgespannt sind, dass sie bei jeder Kraftausübung in Richtung auf den Basiskörper 1 zu in die abstehende Ausgangsposition zurückfedern. Dies kann baulich beispielsweise dadurch sichergestellt werden, dass die Basis eines jeden Verankerungsmittels am Montagepunkt verbreitert ist oder jedes Verankerungsmittel beispielsweise konisch vom Montagepunkt aus Richtung dem Basiskörper abgewandten Ende des Verankerungsmittels verläuft. Die Verankerungsmittel 4 und 7 erfüllen die Aufgabe, eine Befestigung in einem Gewebe sicherzustellen. Vorzugsweise sind ein bis vier erste und zweite Verankerungsmittel so verteilt, dass sie eine Verankerung des implantierbaren medizinischen Gerätes leicht hergestellt werden kann. Besonders bevorzugt sind jeweils drei erste und zweite Verankerungsmittel vorgesehen.

Figur 1 sind auch die erfindungsgemäßen erste und zweite flexiblen Vorrichtungen 5 und 8 zum Zurückziehen jeweils eines zugeordneten ersten und zweiten Verankerungsmittels 4 und 7 zu entnehmen. In diesem Fall sind die flexible Vorrichtungen 5 und 8 als flexible Rippen ausgeführt. Jede der flexiblen Rippen ist auf der Strecke zwischen den jeweiligen ersten Befestigungspunkten 5a, 8a und den Montagepunkten 4a, 7a der zugeordneten Verankerungsmittel 4 und 7 ununterbrochen mit dem Basisköper 1, und zwischen den Befestigungspunkten 5b, 8b und den Montagepunkten 4a, 7a ununterbrochen mit dem jeweiligen zugeordneten ersten und zweiten Verankerungsmittel 4, 7 befestigt. Vorstellbar sind anstatt flexibler Rippen auch Membrane - aufgebaut wie die aus der Herpetofauna bekannten "Schwimmhäute".

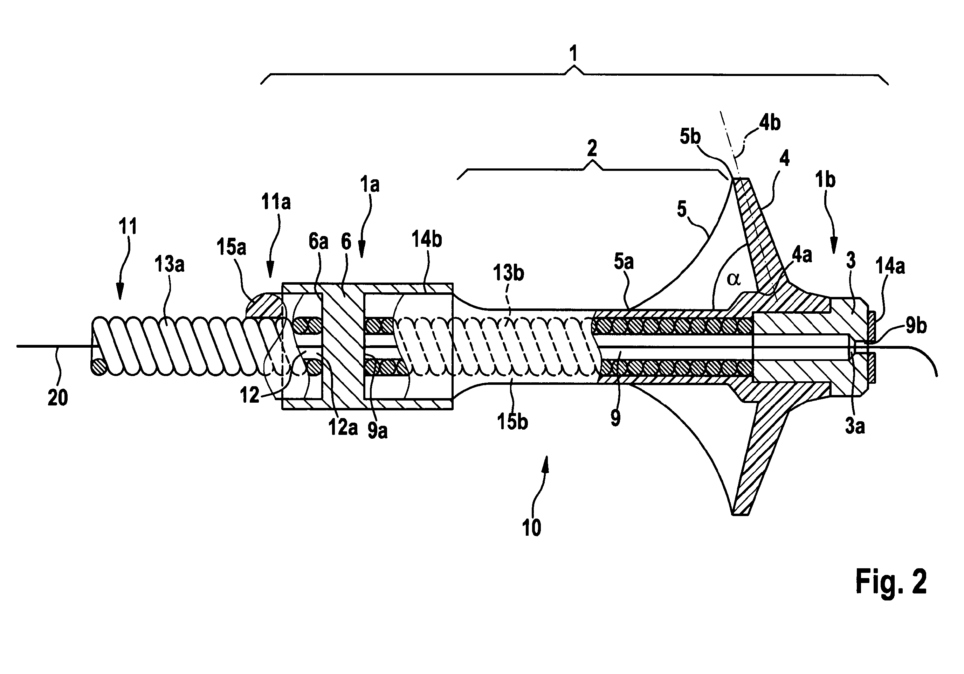

Figur 2 zeigt eine erfindungsgemäße Elektrodenleitung 10 mit einem lang gestreckten Schaft 11 und einer erfindungsgemäßen Befestigungsvorrichtung. Der Schaft 11 hat ein nicht dargestelltes proximales Ende, an dem ein Steckkontakt eine Verbindung zu einem elektromedizinischen Implantat ermöglicht. Der Schaft wird von seinem proximalen Ende bis zu seinem distalen Ende von einem Führungsdrahtlumen 12 durchzogen. Dieses Lumen 12 bildet eine proximale Öffnung im Steckkontakt und eine distale Öffnung 12a am distalen Ende 11a des Schaftes 11. Die Wand des Führungsdrahtlumens 12 wird dabei von einem radial und longitudinal stabilen undehn- oder streckbaren Stabilisierungsmittel 13a gebildet. Im Falle der Elektrodenleitung aus Figur 2 handelt es sich um eine helixförmige Feder, die von der Steckverbindung am proximalen Ende zum distalen Ende 11a des Schaftes 11 verläuft. Das Stabilisierungsmittel 13a ist mit einer außenliegenden isolierenden und gleitfähigen Schicht 15a umgeben, welche bevorzugt aus einem Polymer, besonders bevorzugt aus Polyurethan oder einem Polysiloxan-Copoymer besteht.

Am distalen Ende 11a des Schaftes ist die erfindungsgemäße Befestigungsvorrichtung fest verbunden. Die Befestigungsvorrichtung weist einen Basiskörper 1 mit einem proximalen Ende 1a und einem distalen Ende 1b auf. Weiterhin besteht der Basiskörper aus einem dehnbaren und flexiblen Abschnitt 2 und einem distalen Widerlager 3 am Ende 1b und einem proximalen Widerlager 6 am proximalen Ende 1a des Basiskörpers 1 und wird von einem Führungsdrahtlumen 9 durchzogen, welches im distalen Widerlager 3 eine distale Öffnung 9b und im proximalen Widerlager 6 eine proximale Öffnung 9a bildet. Dabei steht die proximale Öffnung 9a im proximalen Widerlager 6 in direktem Kontakt zur distalen Öffnung 12a des Führungsdrahtlumens 12 des Schaftes 11, so dass ein Führungsdraht 20 vom proximalen Ende des Schaftes 11 durch die Führungsdrahtlumina 9 und 11 und durch distale Öffnung 9b geführt werden kann. So kann die Elektrode an einem Führungsdraht 20 entlang bis zu dem gewünschten Implantationsort der Elektrode geführt werden.

Die Wand des Führungsdrahtlumens 9 wird dabei von einem radial stabilen, aber longitudinal dehn- und/oder streckbaren Stabilisierungsmittel 13b gebildet, in diesem Fall handelt es sich um eine helixförmige Feder, die sich vom proximalen Widerlager 6 zum distalen Widerlager 3 verläuft. Das Stabilisierungsmittel 13b ist mit einer außenliegenden isolierenden und gleitfähigen, ebenfalls longitudinal dehn- und streckbaren Schicht 15b umgeben, welche bevorzugt aus einem Polymer, besonders bevorzugt aus Polyurethan oder einem Polysiloxan-Copoymer, besteht.

Die helixförmige Feder 13b dient gleichzeitig als elektrischer Leiter, der zusammen mit der helixförmigen Feder 13a des Schaftes 11 einen elektrischen Leiter bildet. Dieser elektrische Leiter stellt eine elektrische Verbindung zwischen dem elektromedizinischen Gerät am proximalen Ende und einer elektrisch aktiven Fläche 14a her. Die elektrisch aktive Fläche 14a dient der Abgabe eines im elektromedizinischen Gerät generierten Stimulationsimpulses an das umgebende Gewebe. Die elektrisch aktive Oberfläche 14a befindet sich auf dem distalen Widerlager 3 und hat beispielsweise eine Ringform um die distale Öffnung 9b herum. Zusätzlich kann auch eine weitere elektrisch aktive Oberfläche 14b auf dem proximalen Widerlager 6 vorgesehen werden, um beispielsweise eine bipolare Stimulation zu ermöglichen. In diesem Falle werden zwei elektrische Leiter benötigt, welche beispielsweise durch zwei gegeneinander isolierte und ineinander verschleifte helixförmige Federn bereitgestellt werden.

Auf den Widerlagern 3 und 6 befinden sich Fixierungsmittel 3a und 6a. Bei der erfindungsgemäßen Elektrodenleitung aus Figur 2 wird das Fixierungsmittel 3a durch einen Absatz in der Öffnung 9b gebildet, während das Fixierungsmittel 6a durch im Widerlager 6 durch einen Gegenanschlag gebildet wird, der unmittelbar mit dem Schaft 11 verbunden ist und somit mittelbar vom proximalen Ende aus eine Wirkung oder Kraft auf das Fixiermittel ausgeübt werden kann.

Des Weiteren weist die Vorrichtung in Figur 2 mindestens ein distales flexibles Verankerungsmittel 4 auf, welches am Montagepunkt 4a am distalen Widerlager 3 befestigt ist. Das mindestens eine Verankerungsmittel 4 ist so angeordnet, dass die Längsachse 4b des Verankerungsmittels 4 einen veränderlichen Winkel α zum Basiskörper 1 aufweist. Die Winkel α ist dabei so angelegt, dass das dem Basiskörper 1 abgewandte Ende des Verankerungsmittels 4 in Richtung des proximalen Widerlagers 6 weist. Das mindestens eine Verankerungsmittel 4 ist so konstruiert und vorgespannt, dass es bei jeder Kraftausübung in Richtung auf den Basiskörper 1 in die abstehende Ausgangsposition zurückfedert. Dies kann baulich beispielsweise dadurch sichergestellt werden, dass die Basis des mindestens einen Verankerungsmittels am Montagepunkt 4a verbreitert ist oder jedes Verankerungsmittel beispielsweise konisch vom Montagepunkt aus Richtung des dem Basiskörper abgewandten Endes des Verankerungsmittels 4 verläuft. Das Verankerungsmittel 4 erfüllt die Aufgabe, eine Befestigung in einem Gewebe sicherzustellen. Vorzugsweise sind ein bis vier distale Verankerungsmittel 4 so verteilt, dass eine Verankerung des implantierbaren medizinischen Gerätes leicht hergestellt werden kann. Besonders bevorzugt sind jeweils drei distale Verankerungsmittel 4 vorgesehen.

Jede der distalen Verankerungsmittel 4 ist mit einer erfindungsgemäßen flexiblen Vorrichtung 5 zum Zurückziehen des jeweils zugeordneten distalen Verankerungsmittels 4 verbunden. In diesem Ausführungsbeispiel sind die flexiblen Vorrichtungen als flexible Rippen ausgeführt. Jede der flexiblen Rippen ist auf der Strecke zwischen einem jeweiligen ersten Befestigungspunkt 5a auf dem flexiblen und dehnbaren Abschnitt 2 und dem Montagepunkt 4a des zugeordneten Verankerungsmittels 4 ununterbrochen mit dem Basiskörper 1, und zwischen dem Befestigungspunkt 5b auf dem distalen Verankerungsmittel 4 und dem Montagepunkt 4a ununterbrochen mit dem jeweils zugeordneten distalen Verankerungsmittel 4 befestigt. Vorstellbar sind anstatt flexibler Rippen auch Membrane - aufgebaut wie die aus der Herpetofauna bekannten "Schwimmhäute".

In einer weiteren, nicht ausgeführten Ausführung ist es auch möglich, am proximalen Widerlager 6 Verankerungsmittel vorzusehen, so dass die Form und Wirkung der Ausführungsform aus Figur 1 ähnlich ist.

Figur 1 zeigt die Befestigungsvorrichtung und die Figur 2 eine Elektrodenleitung mit einer solchen Befestigungsvorrichtung in einem ersten Zustand. In diesem Zustand ist der dehnbare flexible Abschnitt 2 des Basiskörpers 1 in einem ungedehnten Zustand. Das heißt, die nicht dehnbaren Rippen 5 und 8 sind in einem entspannten Zustand. Durch ihre konstruktionsbedingte Vorspannung stehen die flexiblen und fingerförmigen Verankerungsmittel 4 und 8 nach außen ab. Mit diesen kann ein dauerhaft oder vorübergehend implantierbares medizinisches Gerät im Gewebe eines menschlichen Körpers verankert werden.

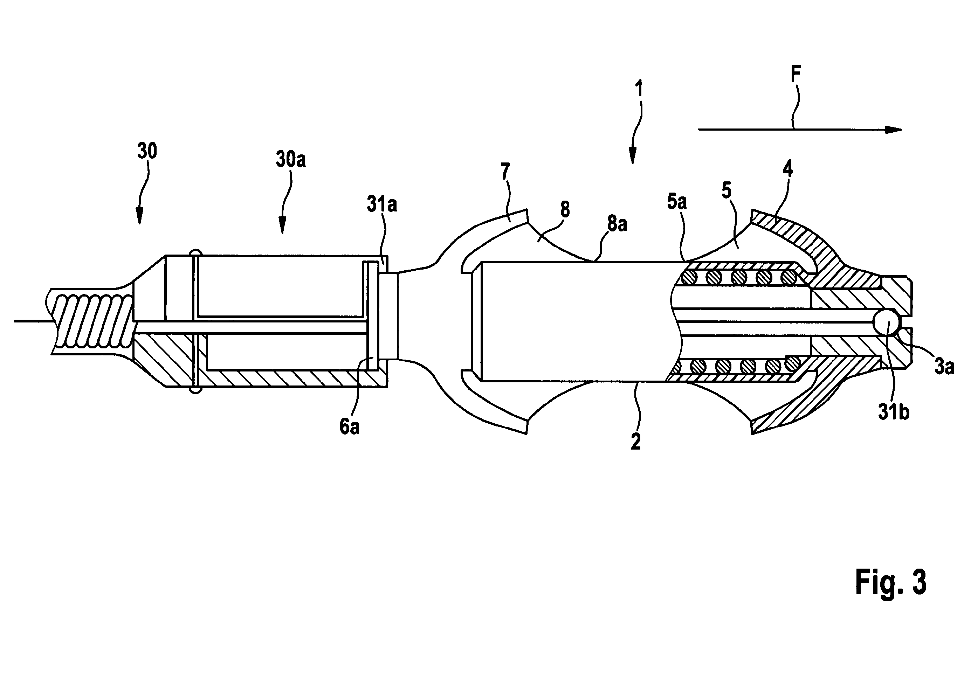

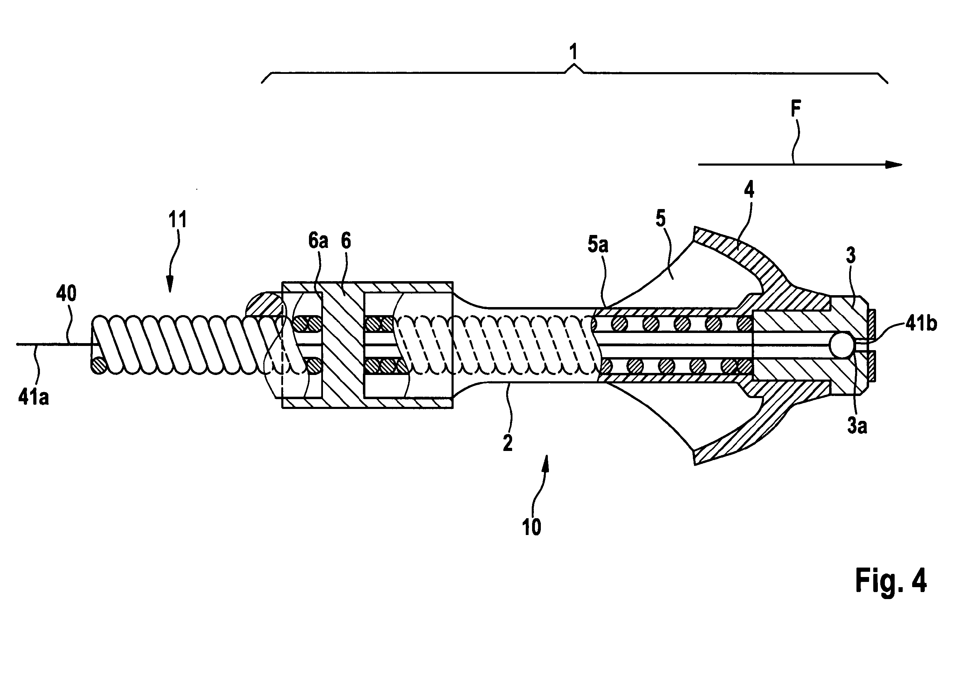

Die Figuren 3 und 4 sollen die Funktionsweise der erfindungsgemäßen Befestigungsvorrichtung zeigen.

Figur 3 zeigt ein erfindungsgemäßes System zum Überführen des dehnbaren Abschnittes 2 einer Befestigungsvorrichtung von einem ersten ungedehnten in einen zweiten gedehnten, gegenüber dem ersten Zustand verlängerten Zustand. Die Vorrichtung ist mit der in Figur 1 beschriebenen identisch, jedoch werden die elektrisch aktiven Elemente nicht dargestellt, weil diese für die Funktionsweise unerheblich sind.

Zusätzlich zu der erfindungsgemäßen Vorrichtung ist in Figur 3 eine Stoßvorrichtung 30 dargestellt, welche ein nicht dargestelltes proximales Ende außerhalb des Körpers und ein distales Ende 30a, an welchem die erfindungsgemäße Befestigungsvorrichtung angebracht ist, zeigt. Die Befestigungsvorrichtung wird über einen zweiten Gegenanschlag 31 a lösbar mit der Stoßvorrichtung verbunden, so dass die Befestigungsvorrichtung zusammen mit der Stoßvorrichtung 30 in den Körper eingebracht werden kann und dort wieder entfernt werden kann. Daher ist der zweite Gegenanschlag 31 a in der dargestellten Ausführungsform als Klemmvorrichtung ausgestaltet und mit dem zweiten Widerlager 6a, welches als Kragen ausgeformt ist, lösbar verbunden.

Weiterhin umfasst die Stoßvorrichtung 30 einen ersten Gegenanschlag 31b, der in direkten Kontakt mit dem ersten Fixierungsmittel 3a der Befestigungsvorrichtung tritt. Der Gegenanschlag 31 b ist an einem gegenüber dem ersten Gegenanschlag 31a verschiebbaren Draht befestigt. Der Gegenanschlag 31 b kann in eine Richtung verschoben werden, die vom proximalen Ende der Stoßvorrichtung 30 und vom zweiten Gegenanschlag 31a weg führt und dabei eine Kraft F auf das erste Fixierungsmittel 3a ausübt, welche parallel zur Bewegungsrichtung des Gegenanschlags und weg vom proximalen Ende der Stoßvorrichtung 30 und des zweiten Gegenanschlags 31 a wirkt. Gleichzeitig bildet der zweite Gegenanschlag ein Auflager, welches eine Kraft bildet, die zur auf das erste Fixierungsmittel 3a wirkenden Kraft entgegengerichtet ist. Somit tritt die Stoßvorrichtung 30 über den Gegenanschlag 31 b und den zweiten Gegenanschlag 31 a in unmittelbare Wechselwirkung mit der Befestigungsvorrichtung, wobei die Kraft direkt auf den dehnbaren und flexiblen Abschnitt 2 wirkt und bewirkt, dass dieser sich dehnt und in einen zweiten gedehnten Zustand überführt wird, welcher gegenüber dem ersten ungedehnten Zustand verlängert ist.

Die Verlängerung des dehnbaren und flexiblen Abschnittes 2 wirkt sich auch auf das mindestens eine erste und zweite fingerförmige Verankerungsmittel 4 und 7 aus. Die Verankerungsmittel 4 und 7 sind über die flexiblen undehnbaren Vorrichtungen zum Zurückziehen 5 und 8 - welche hier als flexible Rippen ausgelegt sind - mit dem dehnbaren und flexiblen Abschnitt 2 fest verbunden. Durch die Dehnung in den zweiten gedehnten und gegenüber dem ersten Zustand verlängerten Zustand wirken die anliegenden Kräfte auf die ersten Befestigungspunkte 5a und 8a der flexiblen Rippen 5 und 8 und übertragen sich dadurch auf die fingerförmigen Verankerungsmittel 4 und 7. Das heißt mit anderen Worten, die Längenänderung des dehnbaren Abschnittes 2 wird über die flexiblen Rippen 5 und 8 auf die Verankerungsmittel 4 und 7 übertragen, wodurch bewirkt wird, dass die Verankerungsmittel 4 und 7 abklappen. Das heißt, dass die im ersten ungedehnten Zustand flachen Winkel α und β in einen spitzen Winkel überführt werden, der sich einstellt, wenn der Abschnitt 2 in einen zweiten gedehnten und gegenüber dem ersten Zustand verlängerten Zustand überführt wird.

Der zweite Zustand ist dann erreicht, wenn die fingerförmigen Verankerungselemente 4 und 7 am Basiskörper 1 anliegen. In diesem Zustand kann die Position des Gegenanschlags 31 b bezüglich des zweiten Gegenanschlages 31 a am proximalen, außerhalb des Körpers liegenden Ende der Stoßvorrichtung 30 fixiert werden. So kann die Befestigungsvorrichtung und damit das dauerhaft oder vorübergehend implantierbare Gerät leicht implantiert, explantiert oder im Körper repositioniert werden. Ist die geeignete Position im Körper gefunden, so kann die Fixierung aufgelöst werden, die Kraft, die auf das erste Fixierungsmittel 3a wirkt, nimmt ab und der dehnbare flexible Abschnitt 2 nimmt seine ursprüngliche ungedehnte Länge ein. Die Abnahme der Kraft auf das erste Fixierungsmittel 3a wird auch auf die flexiblen Rippen 5 und 8 übertragen. Dadurch können die Verankerungsmittel 4 und 7 in die abstehende Ausgangsposition zurückfedern. In diesem Zustand verankern sich die Befestigungsvorrichtung und damit das dauerhaft oder vorübergehend implantierbare Gerät. Nach der erfolgten Verankerung der Befestigungsvorrichtung kann die Verbindung zur Stoßvorrichtung 30 gelöst werden, indem der als Klemmvorrichtung ausgelegte zweite Gegenanschlag 31a gelöst wird.

Figur 4 stellt das erfindungsgemäße System dar, wobei sich der dehnbare und flexible Abschnitt der Elektrodenleitung 10 in einem zweiten gedehnten Zustand befindet. Die Wirkung des Überführens des dehnbaren und flexiblen Abschnittes 2 des Basiskörpers 1 von einem ersten ungedehnten in einen zweiten gedehnten und gegenüber dem ersten Zustand verlängerten Zustand über die undehnbare flexible Vorrichtung zum Zurückziehen 5 auf das mindestens eine distale Verankerungsmittel 4 erfolgt wie in der Figurenbeschreibung zu Figur 3 beschrieben.

Im Unterschied zu der Stoßvorrichtung für die erfindungsgemäße Befestigungsvorrichtung für dauerhaft oder vorübergehend implantierbare Geräte besteht die Stoßvorrichtung 40 für die erfindungsgemäße Elektrodenleitung 10 aus einem Draht 41 a mit einem an dessen distalen Ende montierten Gegenanschlag 41 b, der unmittelbar in Kontakt mit dem in der Öffnung 9a des Führungsdrahtlumens 9 liegenden distalen Fixierungsmittel 3a im distalen Widerlager 3 des Basiskörpers 1 in Kontakt und damit unmittelbar in Wechselwirkung mit dem Basiskörper 1 tritt. Der zweite Gegenanschlag der Stoßvorrichtung 40 wird durch eine nicht dargestellte Klemmvorrichtung am proximalen Ende der Stoßvorrichtung gebildet, welche sich außerhalb des Körpers befindet. Diese Klemmvorrichtung wird mit der Steckverbindung am proximalen Ende der Elektrodenleitung 10 oder mit dem Schaft 11 lösbar verbunden. Der Gegenanschlag tritt damit mittelbar mit dem proximalen Fixierungsmittel 6a am proximalen Widerlager 6 des Basiskörpers 1 in Kontakt und damit mittelbar in Wechselwirkung mit dem Basiskörper 1. Somit kann die Wirkung des zweiten Gegenanschlags als Auflager mittelbar über den Schaft 11 an das proximale Fixierungsmittel 6a übertragen werden und die flexiblen Verankerungsmittel 4 durch Überführen des dehnbaren und flexiblen Abschnittes 2 von einem ungedehnten ersten Zustand in einen gedehnten, gegenüber dem ersten Zustand verlängerten Zustand in Richtung des Basiskörpers bewegt werden.

## Patentansprüche

1. Vorrichtung zur Befestigung von vorübergehend oder dauerhaft implantierbaren medizinischen Geräten, umfassend:
- einen Basiskörper (1) mit einem ersten Ende (1a), einem zweiten Ende (1b) und einem zwischen dem ersten und zweiten Ende befindlichen flexiblen und dehnbaren Abschnitt (2), der von einem ersten ungedehnten in einen zweiten gedehnten und gegenüber dem ersten Zustand verlängerten Zustand überführbar ist,
- ein das zweite Ende (1b) des Basiskörpers bildendes, am Abschnitt (2) fest angebrachtes, unflexibles und undehnbares erstes Widerlager (3)
- mindestens ein erstes flexibles fingerförmiges Verankerungsmittel (4), welches vom Basiskörper (1) absteht und an einem Montagepunkt (4a) am ersten Widerlager (3) angebracht ist, und
- mindestens eine erste flexible/Vorrichtung zum Zurückziehen (5) jeweils eines zugeordneten ersten Verankerungsmittels (4), wobei die Vorrichtung (5) fest an einem ersten Befestigungspunkt (5a) mit dem flexiblen und dehnbaren Abschnitt (2) des Basiskörpers (1) und mit dem zugeordneten ersten Verankerungsmittel (4) verbunden ist, wobei sich beim Überführen des Abschnittes (2) vom ersten in den zweiten Zustand
■ der erste Befestigungspunkt (5a) vom Montagepunkt (4a) entfernt und
■ die erste flexible Vorrichtung zum Zurückziehen (5) nicht ausdehnt,
so dass das zugeordnete erste Verankerungsmittel (4) in Richtung des Basiskörpers (1) bewegt wird,
**dadurch gekennzeichnet, dass**
der flexible und dehnbare Abschnitt (2) und das erste (4) Verankerungsmittel aus demselben dehnbaren Material einstückig hergestellt sind und das Material das erste Widerlager (3) mindestens abschnittsweise umhüllt.

2. Vorrichtung nach Anspruch 1, **dadurch gekennzeichnet, dass** der Basiskörper (1) ein zweites, am ersten Ende (1a) des Basiskörpers fest angebrachtes unflexibles und undehnbares Widerlager (6) aufweist.

3. Vorrichtung nach Anspruch 2, **dadurch gekennzeichnet, dass** sie weiter umfasst:
- mindestens ein zweites flexibles fingerförmiges Verankerungsmittel (7), welches vom Basiskörper (1) absteht und an einem Montagepunkt (7a) am zweiten Widerlager (6) angebracht ist, und
- mindestens eine zweite flexible, undehnbare Vorrichtung zum Zurückziehen (8) jeweils eines zugeordneten zweiten Verankerungsmittels (7), wobei die zweite Vorrichtung (8) fest mit dem flexiblen und dehnbaren Abschnitt (2) des Basiskörpers (1) und mit dem zugeordneten zweiten Verankerungsmittel (7) verbunden ist.

4. Vorrichtung nach einem der Ansprüche 1 bis 3, **dadurch gekennzeichnet, dass** die erste und/oder zweite Vorrichtung zum Zurückziehen (5, 8) einen zweiten Befestigungspunkt (5b, 8b) an dem dem Basiskörper (1) abgewandten Ende des ersten und/oder zweiten Verankerungsmittels (4, 8) aufweist.

5. Vorrichtung nach einem der Ansprüche 1 bis 4, **dadurch gekennzeichnet, dass** die erste und/oder zweite Vorrichtung zum Zurückziehen (5, 8) als dünne, flexible Rippen ausgebildet sind, welche sich zwischen den jeweiligen ersten und zweiten Befestigungspunkten (5a, 5b, 8a, 8b) und den jeweiligen Montagepunkten (4a, 7a) aufspannen und vorzugsweise auf den Strecken zwischen den jeweiligen ersten Befestigungspunkten (5a, 8a) und den jeweiligen Montagepunkten (4a, 7a) und zwischen den jeweiligen zweiten Befestigungspunkten (5b, 8b) und den jeweiligen Montagepunkten (4a, 7a) ununterbrochen am Basiskörper (1) bzw. den ersten und/oder zweiten Verankerungsmitteln (4, 7) befestigt sind.

6. Vorrichtung nach einem der Ansprüche 2 bis 5, **dadurch gekennzeichnet, dass** der dehnbare Abschnitt (2) und das erste und zweite Verankerungsmittel (4, 7) aus demselben dehnbaren Material einstückig hergestellt sind und das Material das erste und zweite Widerlager (3, 6) mindestens abschnittsweise umhüllt.

7. Vorrichtung nach einem der Ansprüche 1 bis 6, **dadurch gekennzeichnet, dass** das erste und/oder zweite Widerlager (3, 6) erste und/oder zweite Fixierungsmittel (3a, 6a) umfasst.

8. Vorrichtung nach Anspruch 1, **dadurch gekennzeichnet, dass** der Basiskörper (1) ein Lumen (9) umfasst, welches eine Öffnung (9a) im ersten Ende (1 a) des Basiskörpers aufweist, sich bis zum zweiten Ende (1 b) erstreckt und vorzugsweise eine Öffnung (9b) im zweiten Ende (1 b) des Basiskörpers aufweist, wobei vorzugsweise die Öffnung (9b) am zweiten Ende (1 b) des Basiskörpers (1) und/oder die Öffnung (9a) des ersten Endes (1a) des Basiskörpers ein Dichtelement, besonders bevorzugt einen Lippendichtring, umfasst.

9. Vorrichtung nach einem der Ansprüche 1 bis 8, **dadurch gekennzeichnet, dass** ein erstes Fixierungsmittel (3a) in der Öffnung (9b) im zweiten Ende (1b) angeordnet ist und vorzugsweise ein zweites Fixierungsmittel (6a) in der Öffnung (9a) des ersten Endes (1a) angeordnet ist.

10. Elektrodenleitung (10), welche umfasst:
- einen lang gestreckten Schaft (11) mit einem proximalen und einem distalen Ende (11a),
- eine am distalen Ende (11a) des Schaftes befestigte Vorrichtung nach einem der Ansprüche 1 bis 9, wobei der Basiskörper (1) eine lang gestreckte, schaftartige Form aufweist und das erste Ende (1a) mit dem distalen Ende (11a) des Schaftes (11) fest verbunden ist,
- mindestens einer elektrisch aktiven Fläche (14a, 14b) am ersten Widerlager und/oder zweiten Widerlager (3, 6), und
- mindestens einem elektrischen Leiter, der sich vom proximalen Ende des Schaftes über das distale Ende des Schaftes hinaus bis zu den elektrisch aktiven Flächen erstreckt.

11. Elektrodenleitung nach Anspruch 10, **dadurch gekennzeichnet, dass** der Schaft weiterhin umfasst:
- ein Lumen (12), welches sich vom proximalen Ende bis in das distale Ende (11a) des Schaftes erstreckt und im proximalen und distalen Ende (11a) eine Öffnung bildet, wobei die Öffnung im distalen Ende (11a) und die Öffnung (9a) des Basiskörpers (1) verbunden sind derart, dass ein Führungsdraht und/oder Mandrin (20) geführt werden kann, um die Elektrodenleitung zu führen und/oder den flexiblen dehnbaren Abschnitt (2) zu strecken und/oder zu dehnen.

12. System zum Überführen des dehnbaren Abschnittes (2) einer Vorrichtung oder Elektrodenleitung nach einem der Ansprüche 1 bis 11 von einem ersten ungedehnten in einen zweiten gedehnten, gegenüber dem ersten Zustand verlängerten Zustand, umfassend:
- eine Vorrichtung oder Elektrodenleitung nach einem der Ansprüche 1 bis 11, wobei das erste Widerlager (3) ein erstes Fixierungsmittel (3a) aufweist und
- eine Stoßvorrichtung (30, 40) mit einem ersten Gegenanschlag (31 b, 41 b), der unmittelbar in Wechselwirkung mit dem ersten Fixierungsmittel (3a) tritt.

13. System nach Anspruch 12, **dadurch gekennzeichnet, dass** die Stoßvorrichtung (30, 40) einen zweiten Gegenanschlag (31 a) umfasst, der mittelbar oder unmittelbar in Wechselwirkung mit dem zweiten Fixierungsmittel (6a) tritt.

14. System nach Anspruch 13, **dadurch gekennzeichnet, dass** der zweite Gegenanschlag ein Klemmmittel ist, mit dem die Stoßvorrichtung (30, 40) mit dem Schaft (11) der Elektrodenleitung fixiert werden kann und somit in mittelbare Wechselwirkung über den Schaft (11) auf das zweite Fixierungsmittel (6a) tritt, um so ein Auflager bezüglich der Wechselwirkung des ersten Gegenanschlags (41 b) mit dem ersten Fixierungsmittel (3a) zu bilden.

## Claims

1. An apparatus for attaching temporarily or permanently implantable medical devices, comprising:
- a base body (1), having a first end (1 a), a second end (1b) and a flexible and expandable section (2) which is located between the first and second ends and which can be transferred from a first non-expanded state to a second expanded state which is extended compared to the first state,
- a non-flexible and non-expandable first abutment (3), which is rigidly attached to the section (2) and forms the second end (1 b) of the base body,
- at least one first flexible finger-shaped anchoring means (4), which projects from the base body (1) and is provided at a mounting point (4a) on the first abutment (3), and
- at least one first flexible apparatus for retracting (5) a respective associated first anchoring means (4), wherein at a first attachment point (5a) the apparatus (5) is rigidly connected to the flexible and expandable section (2) of the base body (1) and to the associated first anchoring means (4), wherein when transferring the section (2) from the first to the second state
• the first attachment point (5a) moves away from the mounting point (4a), and
• the first flexible apparatus for retracting (5) does not expand, so that the associated first anchoring means (4) is moved in the direction of the base body (1),
**characterized in that**
the flexible and expandable section (2) and the first (4) anchoring means are integrally produced from the same expandable material and the material envelopes the first abutment (3) at least in some sections.

2. The apparatus according to claim 1, **characterized in that** the base body (1) comprises a second non-flexible and non-expandable abutment (6) which is rigidly provided at the first end (1 a) of the base body.

3. The apparatus according to claim 2, further comprising:
- at least one second flexible finger-shaped anchoring means (7), which projects from the base body (1) and is provided at a mounting point (7a) on the second abutment (6), and
- at least one second flexible non-expandable apparatus for retracting (8) a respective associated second anchoring means (7), wherein the second apparatus (8) is rigidly connected to the flexible and expandable section (2) of the base body (1) and to the associated second anchoring means (7).

4. An apparatus according to any one of claims 1 to 3, **characterized in that** the first and/or second apparatuses for retracting (5, 8) comprise a second attachment point (5b, 8b) at the end of the first and/or second anchoring means (4, 8) which is spaced away from the base body (1).

5. An apparatus according to any one of claims 1 to 4, **characterized in that** the first and/or second apparatuses for retracting (5, 8) are designed as thin, flexible ribs, which stretch between the respective first and second attachment points (5a, 5b, 8a, 8b) and the respective mounting points (4a, 7a) and which, preferably on the segments between the respective first attachment points (5a, 8a) and the respective mounting points (4a, 7a), and between the respective second attachment points (5b, 8b) and the respective mounting points (4a, 7a), are attached in an uninterrupted manner to the base body (1) or the first and/or second anchoring means (4, 7).

6. An apparatus according to any one of claims 2 to 5, **characterized in that** the expandable section (2) and the first and second anchoring means (4, 7) are integrally produced from the same expandable material and the material envelopes the first and second abutments (3, 6) at least in some sections.

7. An apparatus according to any one of claims 1 to 6, **characterized in that** the first and/or second abutments (3, 6) comprise first and/or second fixing means (3a, 6a).

8. The apparatus according to claim 1, **characterized in that** the base body (1) comprises a lumen (9), which has an opening (9a) in the first end (1 a) of the base body, extends to the second end (1b) and preferably has an opening (9b) in the second end (1 b) of the base body, wherein preferably the opening (9b) at the second end (1 b) of the base body (1) and/or the opening (9a) of the first end (1 a) of the base body comprises a sealing element, with a lip sealing ring being particularly preferred.

9. An apparatus according to any one of claims 1 to 8, **characterized in that** a first fixing means (3a) is disposed in the opening (9b) in the second end (1b) and preferably a second fixing means (6a) is disposed in the opening (9a) of the first end (1 a).

10. An electrode lead (10), comprising:
- an elongated shaft (11) having a proximal and a distal end (11a),
- an apparatus according to any one of claims 1 to 9, which is attached at the distal end (11 a) of the shaft, wherein the base body (1) has an elongated, shaft-like shape and the first end (1a) is rigidly connected to the distal end (11a) of the shaft (11),
- at least one electrically active surface (14a, 14b) at the first abutment and/or second abutment (3, 6), and
- at least one electrical conductor, which extends from the proximal end of the shaft beyond the distal end of the shaft up to the electrically active surfaces.

11. The electrode lead according to claim 10, **characterized in that** the shaft further comprises:
- a lumen (12), which extends from the proximal end into the distal end (11a) of the shaft and forms a respective opening in the proximal and distal ends (11a), wherein the opening in the distal end (11a) and the opening (9a) of the base body (1) are connected such that a guide wire and/or a mandrin (20) can be guided so as to guide the electrode lead and/or stretch and/or expand the flexible expandable section (2).

12. A system for transferring the expandable section (2) of an apparatus or electrode lead according to any one of claims 1 to 11 from a first non-expanded state to a second expanded state which is extended compared to the first state, comprising:
- an apparatus or electrode lead according to any one of claims 1 to 11, wherein the first abutment (3) comprises a first fixing means (3a), and
- a push apparatus (30, 40) comprising a first counterstop (31b, 41b), which directly interacts with the first fixing means (3a).

13. The system according to claim 12, **characterized in that** the push apparatus (30, 40) comprises a second counterstop (31a), which directly or indirectly interacts with the second fixing means (6a).

14. The system according to claim 13, **characterized in that** the second counterstop is a clamping means, by means of which the push apparatus (30, 40) can be fixed to the shaft (11) of the electrode lead and thus enter into indirect interaction via the shaft (11) with the second fixing means (6a) so as to form a support with regard to the interaction of the first counterstop (41 a) with the first fixing means (3a).

## Revendications

1. Appareil pour attacher de manière temporaire ou permanente des dispositifs médicaux implantables, comprenant :
- un corps de base (1), ayant une première extrémité (1a), une seconde extrémité (1 b) et une section souple et extensible (2) qui est située entre les première et seconde extrémités et qui peut être amenée à passer d'un première état non étendu à un second état étendu qui est étendu par comparaison au premier état,
- une première butée non souple et non extensible (3), qui est attachée de manière rigide à la section (2) et forme la seconde extrémité (1 b) du corps de base,
- au moins un premier moyen d'ancrage en forme de doigt souple (4), qui se projette à partir du corps de base (1) et est disposé à un point de montage (4a) sur la première butée (3), et
- au moins un premier appareil souple pour rétracter (5) un premier moyen d'ancrage (4) associé respectif, l'appareil (5) étant relié de manière rigide en un premier point d'attache à la section souple et extensible (2) du corps de base (1) et au premier moyen d'ancrage (4) associé, dans lequel lors du passage de la section (2) du premier au second état
• le premier point d'attache (5a) s'écarte du point de montage (4a), et
• le premier appareil souple pour rétracter (5) ne s'étend pas,
de telle sorte que le premier moyen d'ancrage (4) associé est déplacé dans la direction du corps de base (1),
**caractérisé par le fait que**
la section souple et extensible (2) et le premier moyen d'ancrage (4) sont produits d'un seul tenant à partir de la même matière extensible et la matière enveloppe la première butée (3) au moins dans certaines sections.

2. Appareil selon la revendication 1, **caractérisé par le fait que** le corps de base (1) comprend une seconde butée non souple et non extensible (6) qui est disposée de manière rigide à la première extrémité (1 a) du corps de base.

3. Appareil selon la revendication 2, comprenant en outre:
- au moins un second moyen d'ancrage en forme de doigt souple (7), qui se projette à partir du corps de base (1) est disposé à un point de montage (7a) sur la seconde butée (6), et
- au moins un second appareil souple non extensible pour rétracter (8) un second moyen d'ancrage (7) associé respectif, le second appareil (8) étant relié de manière rigide à la section souple et extensible (2) du corps de base (1) et au second moyen d'ancrage (7) associé.

4. Appareil selon l'une quelconque des revendications 1 à 4, **caractérisé par le fait que** les premier et/ou second appareils pour rétracter (5, 8) comprennent un second point d'attache (5b, 8b) à l'extrémité des premier et/ou second moyens d'ancrage (4, 8) qui est éloignée du corps de base (1).

5. Appareil selon l'une quelconque des revendications 1 à 4, **caractérisé par le fait que** les premier et/ou second appareils pour rétracter (5, 8) sont agencés sous la forme de nervures minces, souples, qui s'étirent entre les premier et second points d'attache (5a, 5b, 8a, 8b) respectifs et les points de montage (4a, 7a) respectifs et qui, de préférence sur les segments entre les premiers point d'attache (5a, 8a) respectifs et les points de montage (4a, 7a) respectifs, et entre les seconds points d'attache (5b, 8b) respectifs et les points de montage (4a, 7a) respectifs, sont attachées d'une manière ininterrompue au corps de base (1) ou aux premier et/ou second moyens d'ancrage (4, 7).

6. Appareil selon l'une quelconque des revendications 2 à 5, **caractérisé par le fait que** la section extensible (2) et les premier et second moyens d'ancrage (4, 7) sont produits d'un seul tenant à partir de la même matière extensible et la matière enveloppe les première et seconde butées (3, 6) au moins dans certaines sections.

7. Appareil selon l'une quelconque des revendications 1 à 6, **caractérisé par le fait que** les première et/ou seconde butée (3, 6) comprennent des premier et/ou second moyens de fixation (3a, 6a).

8. Appareil selon la revendication 1, **caractérisé par le fait que** le corps de base (1) comprend une lumière (9), qui a une ouverture (9a) dans la première extrémité (1a) du corps de base, s'étend jusqu'à la seconde extrémité (1b) et de préférence a une ouverture (9b) dans la seconde extrémité (1 b) du corps de base, l'ouverture (9b) à la seconde extrémité (1b) du corps de base (1) et/ou l'ouverture (9a) de la première extrémité (1 a) du corps de base comprenant de préférence un élément d'étanchéité, une bague d'étanchéité à lèvre étant particulièrement préférée.

9. Appareil selon l'une quelconque des revendications 1 à 8, **caractérisé par le fait qu'**un premier moyen de fixation (3a) est disposé dans l'ouverture (9b) dans la seconde extrémité (1b) et de préférence un second moyen de fixation (6a) est disposé dans l'ouverture (9a) de la première extrémité (1a).

10. Ligne d'électrode (10), comprenant :
- un arbre allongé (11) ayant une extrémité proximale et une extrémité distale (11 a),
- un appareil tel que défini à l'une quelconque des revendications 1 à 9, qui est attaché au niveau de l'extrémité distale (11a) de l'arbre, le corps de base (1) ayant une forme de type arbre, allongée, et la première extrémité (1 a) étant reliée de manière rigide à l'extrémité distale (11 a) de l'arbre (11),
- au moins une surface électriquement active (14a, 14b) au niveau de la première butée et/ou de la seconde butée (3, 6), et
- au moins un conducteur électrique, qui s'étend à partir de l'extrémité proximale de l'arbre, au-delà de l'extrémité distale de l'arbre, jusqu'aux surfaces électriquement actives.

11. Ligne d'électrode selon la revendication 10, **caractérisé par le fait que** l'arbre comprend en outre:
- une lumière (12), qui s'étend à partir de l'extrémité proximale dans l'extrémité distale (11a) de l'arbre et forme une ouverture respective dans les extrémités proximale et distale (11a), l'ouverture dans l'extrémité distale (11a) et l'ouverture (9a) du corps de base (1) étant reliées de telle sorte qu'un fil-guide et/ou un mandrin (20) peuvent être guidés de façon à guider la ligne d'électrode et/ou étirer et/ou étendre la section extensible souple (2).

12. Système pour faire passer la section extensible (2) d'un appareil ou d'une ligne d'électrode tels que définis à l'une quelconque des revendications 1 à 11 d'un premier état non étendu à un second état étendu qui est étendu par comparaison au premier état, comprenant:
- un appareil ou une ligne d'électrode tels que définis à l'une quelconque des revendications 1 à 11, la première butée (3) comprenant un premier moyen de fixation (3a), et
- un appareil de poussée (30, 40) comprenant une première contrebutée (31 b, 41 b), qui interagit directement avec le premier moyen de fixation (3a).

13. Système selon la revendication 12, **caractérisé par le fait que** l'appareil de poussée (30, 40) comprend une seconde contrebutée (31a), qui interagit directement ou indirectement avec le second moyen de fixation (6a).

14. Système selon la revendication 13, **caractérisé par le fait que** la seconde contrebutée est un moyen de serrage, au moyen duquel l'appareil de poussée (30, 40) peut être fixé à l'arbre (11) de la ligne d'électrode et ainsi entrer en interaction directe par l'intermédiaire de l'arbre (11) avec le second moyen de fixation (6a) de façon à former un support en ce qui concerne l'interaction de la première contrebutée (41 a) avec le premier moyen de fixation (3a).
